Europäisches Patentamt

European Patent Office

Office européen des brevets

(11) Publication number: **0 328 275**
**A1**

## EUROPEAN PATENT APPLICATION

(21) Application number: **89300794.8**

(51) Int. Cl.⁴: **G01N 33/18**

(22) Date of filing: **27.01.89**

(30) Priority: **29.01.88 GB 8802069**
**23.07.88 GB 8817589**

(43) Date of publication of application:
**16.08.89 Bulletin 89/33**

(84) Designated Contracting States:
**ES**

(71) Applicant: **SOUTH WEST WATER AUTHORITY**
**Peninsula House Rydon Lane**
**Exeter, EX2 7HR(GB)**

(72) Inventor: **Dale, Bryan**
**8 Canterbury Road Exwick**
**Exeter Devon(GB)**
Inventor: **Lee, Peter George**
**31 Hellings Gardens The Green**
**Broadclyst Exeter Devon, EX5 2DX(GB)**

(74) Representative: **Newby, John Ross et al**
**J.Y. & G.W. Johnson Furnival House 14/18**
**High Holborn**
**London WC1V 6DE(GB)**

(54) **Assaying aluminium in drinking water.**

(57) The aluminium content in a water supply is determined by a method which involves forming the aluminium in a sample stream of the water into a complex with added fluoride and sensing the fluoride content electrically. Equipment for carrying out the invention comprises means (10) to mix a sample stream (in 1) with a standard sodium fluoride solution (in 9), warm it (in 12) and sense the PD generated between electrodes 13 and 14. Solutions of known aluminium concentration can be drawn from reservoirs 3 and 4 to calibrate the fluoride ion selective electrode 14.

FIG.1

## ASSAYING ALUMINIUM IN DRINKING WATER

Technical Field

This invention is concerned with the determination of trace quantities of aluminium in a water supply.

Background Art

The Commission of the European Economic Community has recommended to companies and authorities responsible for local drinking water supplies, that such waters should contain no more than 0.2 milligrams per litre of aluminium: this recommendation is likely to become a regulation by 1990. A considerable proportion of the water industry employs alum as a flocculent for the turbidity of raw water and users of alum are most at risk of infringing the recommendation, but for the entire water industry there remains the problem of continuously assaying the quality of the water released from water treatment works when such works are staffed by analysts, if at all, for only part of the twenty-four hours of each day.

Summary of the Invention

This invention seeks to provide an improved method of and equipment for assaying the aluminium content in drinking water which will reduce and at best solve the aforesaid problem.

According to the present invention an automated continuous method of assaying the aluminium content of water, comprises abstracting a sample stream from a stream of water, flowing an acid buffered standard active fluoride ion solution at a rate which is in constant proportion to the rate of flow of the sample stream, mixing the sample stream of water and the flowing solution to form a mixed liquid, imposing a standard temperature on the mixed liquid, allowing active fluoride ions to form a complex with aluminium in the mixed liquid, flowing the mixed liquid into contact with a calibrated fluoride ion selective electrode and a reference electrode, sensing the potential difference between the said electrodes, and electronically computing the aluminium content of the sample from the sensed potential difference.

Conveniently acid is added to an abstracted stream to bring the aluminium content into solution at a pH of around 2.0 following which the required sample stream is taken and mixed with the buffered fluoride ion solution.

Complexes in accordance with the generic formula

$$[Al(H_2O)_{(6-x)}F_x]^{(3-x)+} \quad (1)$$

are formed when acidic solutions of aluminium and active fluoride ions are mixed. Below the pKa of hydrofluoric acid (3.2), the complex is slow to form and anomalous reductions in the active fluoride ion concentrations arise from the formation of HF. Again, above pH 5.5, hydroxyl ions compete with fluoride ions to complex with aluminium and such ions may also cause a deterioration of the fluoride ion selective electrode. Hence the preferred range of pHs employed in the process is 4.0 to 5.5 and the buffered standard solution is desirably chosen to maintain a narrow band of pH within that range. Preferably the band width is 4.5 to 5.0. The value of the numeral x in the generic formula given above, is determined experimentally, and should be about 2.1 in line with the expected predominance of the species $[Al(H_2O)_4F_2]^+$ at the preferred pH band, but in some waters containing an anion which competes with fluoride in complexing aluminium, the value of x will be less than 2. This result is, first of all, an indication of the presence or incursion of such an ion, which in itself may be useful. Should the concentration of the interfering anion be a constant feature of the water, the experimentally determined value of x will be a constant and may be used in the assay of aluminium. Otherwise the interfering anion can be complexed into an inactive form.

The method of the invention requires a measurable residual active fluoride ion content in the mixed liquid following the formation of the aluminium complex and the linear response of the potential difference between the electrodes to the logarithm of the concentration of active fluoride ions is preserved when the residual concentration is greater than 0.2 milligrams per litre of such fluoride ions: below that concentration the linearity of the relationship is lost and the potential difference sensed no longer gives accurate determinations of aluminium content.

An active ion meter is the preferred instrument for sensing the potential difference appearing between the electrodes and translating the sensed value into a measure of the aluminium content of the sample. In

2

common with some known ion meters, a microprocessor is desirably incorporated to facilitate calibration and calculation of measurement parameters. Where such a microprocessor is present it can be used to control the automatic sequencing of valves in automated equipment provided to operate the method, as will be more fully described hereafter.

The standard temperature imposed on the mixed liquid is preferably above ambient temperature and is thus normally achieved by warming the mixed liquid. The complex forms more rapidly as the temperature rises and, at about 45°C., the period needed to achieve equilibration of the complex is of the order of one minute at the preferred pH band.

The method of the invention preferably utilises periodic recalibration of the ion selective electrode and it is convenient for this recalibration to be carried out automatically. For such recalibration, a stream of a standard solution of aluminium ions can be substituted for the sample stream in the method of the invention and the mixed liquid flow maintained across the electrodes until a steady potential difference is attained. The datum can then be logged in a suitable memory. Repeating the method with a second standard solution of different aluminium content, identifies a second level of potential difference which can also be logged. Using logged data from two such measurements with known aluminium contents enables the slope of the linear relationship of the sensed potential difference to the logarithm of the concentration of active fluoride ions to be determined and the selective electrode is thereby recalibrated.

The reduction of the active fluoride ion content of the mixed liquid due to complexation with aluminium, in the analytic or calibration mode of operation, causes an electropositive change $(-\Delta E)$ in the potential of the fluoride ion selective electrode versus the reference electrode and that change is related to the concentration of aluminium as follows:

$$[Al] = \frac{V_1 + V_2}{V_1 x} \, 10^{\frac{-\Delta E}{k}} \qquad \dots \dots \dots \dots \dots \dots (2)$$

where

[Al] is the concentration of aluminium in the sample stream;

$V_1$ is the volume of the sample stream;

$V_2$ is the volume of the buffered fluoride ion solution;

x is the number of fluoride ions in the complex molecule;

$-\Delta E$ is the electropositive change in the potential of the ion-selective electrode versus the reference electrode showing increases in [Al];

and

k is theoretically $2.303 \frac{RT}{nF}$ but in practice is the experimentally determined slope of the linear E versus log $[F^-]$ plot.

With the exception of the variable $-\Delta E$, the other values on the right-hand side of equation (2) are constants of the equipment and the method, so that equation (2) reduces to

$[Al] = C. -\Delta E \qquad (3)$

where C is a constant.

There are some waters which naturally contain ferric ion and as ferric ion also complexes with fluoride ion, the ferric ion should be rendered inactive before the aluminium determination is carried out. Complexing agents are available which will combine with ferric ion whilst not interfering with aluminium, and one example would be ascorbic acid. Therefore buffered fluoride solutions which also contain a ferric complexing agent are useful in carrying out the method of this invention.

Equally there are waters which either naturally, or as a result of treatment, contain fluoride ions. In such cases it is essential to determine the inherent fluoride ion content by firstly complexing all trivalent metal ions with a non-fluoride complexing agent so that the fluoride content present when the potential difference is assessed consists entirely of the added active fluoride ions.

The invention also extends to automated assaying equipment for carrying out the method, and in this aspect comprises an active ion meter, a reference electrode and a calibrated fluoride ion selective electrode electrically connected via the ion meter, a first conduit adapted to contain the sample stream, a second conduit adapted to conduct the buffered standard active fluoride solution, means to induce a constant rate of flow in each of the first and second conduits, a confluence of the first and second conduits adapted to allow the combination of the sample stream and fluoride ion solution to form a mixed liquid, means to impose a standard temperature on the mixed liquid, means to conduct the mixed liquid into contact with the said electrodes, and means to record the EMF generated between said electrodes as determined by the ion meter.

For the purpose of recalibration of the fluoride ion selective electrode, the equipment preferably also comprises two reservoirs adapted to contain, respectively, first and second standard solutions of an aluminium salt differing in salt concentration, valve means to control the flow of standard solution from each reservoir, a third conduit adapted to receive liquid from the valve means, a confluence of the third conduit with the second conduit, valve means to control the entry of the sample stream into the first conduit and an automatic sequencer adapted to open the valve means in sequence for predetermined periods in such a way that only one of the sample stream, the first and the second standard aluminium salt solutions may flow into the confluence with the second conduit at a time.

It is convenient to use a common conduit for the sample stream and the first and second standard aluminium solutions.

The conduits used in the equipment are preferably of small bore tubing to encourage linear flow therein and to minimise back-mixing. The preferred means to induce a constant rate of flow in each of the first and second conduits, is a peristaltic pump operating on both similarly sized conduits simultaneously. This requires that each conduit, at least over the length in contact with the pump, is made of an elastomeric material.

Suitably the means to impose a standard temperature on the mixed liquid includes a digester coil and one or more reactor coils all maintained at the desired temperature. Conveniently the said means warms the mixed liquid to 45° C for at least five minutes to ensure the equilibration of complex formation and, to this end, it is convenient that the conduit for the mixed liquid should be a helix or other labyrinthine structure within a heated member (e.g. a heated fluid bath).

## Brief Description of the Drawing

The invention will now be more fully described, by way of example, with reference to the accompanying drawing, in which:

Figure 1 is a schematic block diagram of one embodiment of equipment for carrying out the method, and

Figure 2 is a more detailed block diagram of a second embodiment of equipment according to the invention.

## Description of Preferred Embodiments

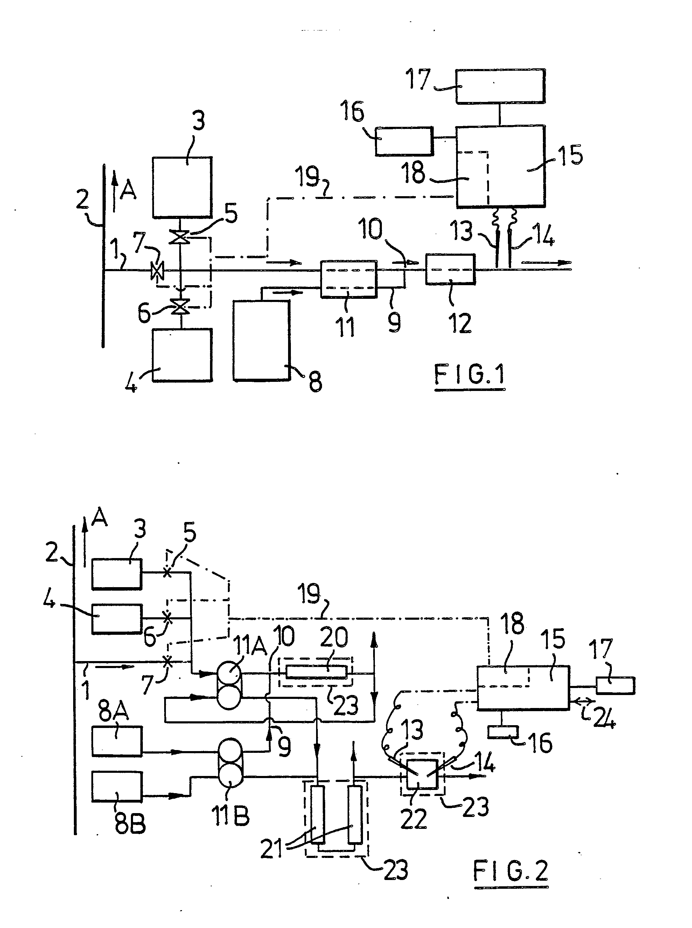

Referring to Figure 1, a conduit 1 is a side branch on a pipe 2 through which water to be sampled is flowing in the direction of the arrow A. Reservoirs 3 and 4 respectively contain different standard solutions of aluminium nitrate $S_1$ and $S_2$ (one may be devoid of aluminium and is then a zero standard solution) and are connected via valves 5 and 6 with the conduit 1. Valve 7 in conduit 1 controls the flow of the stream of sampled water. Reservoir 8 contains a standard sodium fluoride solution, say 5 milligrams of fluoride ions per litre, buffered to a pH of between 4.5 and 5.0 with an acetic acid/sodium acetate buffer. Reservoir 8 is connected via conduit 9 with a confluence 10 where conduits 1 and 9 join. Conduits 1 and 9 pass collaterally through a peristaltic pump 11 upstream of the confluence 10 and as the conduits where they pass through the pump 11 are flexible tubes of identically sized bores, the effect of the pump 11 is to deliver equal volumes of liquids through the conduits 1 and 9 to the confluence 10. Downstream of the confluence 10 the mixed liquid is contained in the conduit 1, passing through a heated block 12, which imposes a temperature of 45° C on the mixed liquid, and into operative contact with a reference electrode 13 and a fluoride ion selective electrode 14, the electrodes 13 and 14 being electrically connected via an active ion meter 15. The active ion meter comprises a millivolt meter, and a microprocessor/random access memory circuit to cope, by known methods, with retaining the datum calibration voltages established experimentally and to carry out the calculation.

$$[Al] = C. -\Delta E$$

from the change $(-\Delta E)$ in the potential difference sensed between the electrodes 13 and 14 occasioned by variations in the aluminium content of the sampled (e.g. drinking) water. The active ion meter 15 is electrically connected to a digital display 16 and a pen recorder 17 on both of which a direct reading of the concentration of aluminium in the sampled water is displayed.

For the convenience of confining the electronic circuitry to one box, the active ion meter 15 can also house a sequencer 18 arranged to control, via connections 19, the scheduled sequential opening and closing of valves 5, 6 and 7 so that only one valve is open at any time.

With the valve 7 open, the equipment is in its analytic mode and the sample stream is metered by the pump 11 into contact with a metered flow of the buffered standard fluoride solution. The mixed liquid thus formed passes through the heated block 12, attaining a temperature of 45°C and shortly thereafter (e.g. five minutes later) comes into operative contact with the electrodes 13 and 14. Changes in the logarithm of concentration of aluminium in the sampled water are directly related to electropositive changes in the EMF appearing between the electrodes 13 and 14 and such changes are translated electronically into a direct read out on the recorder 17 of the aluminium concentration in the sampled water.

In its calibration mode, valve 7 is closed and valve 5 is opened to allow the first standard aluminium solution $S_1$ from reservoir 3 to enter the conduit 1 where within a short interval it displaces the residue of the sample stream. The remainder of the process remains unchanged with the exception that by the time a steady signal characteristic of the mixed calibrating liquid has been established, the correlation of a known aluminium content and the experimentally determined EMF are transferred as a datum to the electronic memory. Closing valve 5 and opening valve 6 allows the process to be repeated with the second standard aluminium solution $S_2$ providing a second datum for transfer to the memory. These data determine the slope, k of the linear relationship set forth in equation 2 above. At the completion of the calibration procedure, valve 6 is closed and valve 7 opened so that the analytic mode is reintroduced.

Figure 2 shows a modified form of equipment and, where appropriate the same reference numerals as have been used in Figure 1 are employed. The main features of difference between the Figure 1 and Figure 2 arrangements are:

Reservoir 8 is divided into a tank 8A for acid and a tank 8B for buffer.

The pump 11 is shown as two linked pumps 11A and 11B.

A digester coil 20, a reactor coil 21 and a cell 22 are immersed in a common heated water bath 23.

An interface output 24 (e.g. to RS 232) is provided on the meter 15.

## Claims

1. An automated continuous method of assaying the aluminium content of water, which comprises abstracting a sample stream from a stream of water, flowing an acid buffered standard active fluoride ion solution at a rate which is in constant proportion to the rate of flow of the sample stream, mixing the sample stream of water and the flowing solution to form a mixed liquid, imposing a standard temperature on the mixed liquid, allowing active fluoride ions to form a complex with aluminium in the mixed liquid, flowing the mixed liquid into contact with a calibrated fluoride ion selective electrode and a reference electrode, sensing the potential difference between the said electrodes, and electronically computing the aluminium content of the sample from the sensed potential difference.

2. A method as claimed in claim 1, characterised in that acid is added to the abstracted stream to bring the aluminium content into solution at a pH of around 2.0 following which the required sample stream is taken and mixed with the buffered fluoride ion solution.

3. A method as claimed in claim 1, characterised in that the pH of the mixed liquid is in the range 4.0 to 5.5.

4. A method as claimed in claim 3, characterised in that the residual active fluoride ion content in the mixed liquid following the formation of the aluminium complex is greater than 0.2 milligrams per litre of such fluoride ions.

5. A method as claimed in claim 4, characterised in that the mixed liquid is warmed to a predetermined temperature prior to the sensing of the potential difference.

6. A method as claimed in claim 5, characterised in that the ion selective electrode is periodically calibrated using a first standard solution of aluminium ions substituted for the sample stream to give a first recalibration potential difference and then a second standard solution of different aluminium content to give a second recalibration potential difference, data from said two measurements with known aluminium contents enabling the selective electrode to thereby be recalibrated.

7. A method as claimed in claim 5, characterised in that a buffered fluoride solution which also contains a ferric complexing agent is used to form the mixed liquid.

8. A method as claimed in claim 5, characterised in that the inherent fluoride ion content of the sample stream is determined by firstly complexing all trivalent metal ions with a non-fluoride complexing agent so that the fluoride content present when the potential difference is assessed consists entirely of the added active fluoride ions.

9. Automated assaying equipment for carrying out the method of claim 1, which comprises an active ion meter, a reference electrode and a calibrated fluoride ion selective electrode electrically connected via the ion meter, a first conduit adapted to contain the sample stream, a second conduit adapted to conduct the buffered standard active fluoride solution, means to induce a constant rate of flow in each of the first and second conduits, a confluence of the first and second conduits adapted to allow the combination of the sample stream and fluoride ion solution to form a mixed liquid, means to impose a standard temperature on the mixed liquid, means to conduct the mixed liquid into contact with the said electrodes, and means to record the EMF generated between said electrodes as determined by the ion meter.

10. Equipment as claimed in claim 9, characterised in that the equipment also comprises two reservoirs adapted to contain, respectively, first and second standard solutions of an aluminium salt differing in salt concentration, valve means to control the flow of standard solution from each reservoir, a third conduit adapted to receive liquid from the valve means, a confluence of the third conduit with the second conduit, valve means to control the entry of the sample stream into the first conduit and an automatic sequencer adapted to open the valve means in sequence for predetermined periods in such a way that only one of the sample stream, the first and second standard aluminium salt solutions may flow into the confluence with the second conduit at a time.

FIG.1

FIG.2

European Patent
Office

**EUROPEAN SEARCH REPORT**

Application Number

EP 89 30 0794

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 524 641 (SOCIETE LYONNAISE DES EAUX) <br> * Page 2, lines 8-26; page 5, claim 1 * | 1 | G 01 N 33/18 |
| A | DE-A-2 505 255 (ALINEGA) <br> * Page 1, paragraph 1; claim 1 * | 1 | |
| A | GB-A-1 574 225 (HOECHST) <br> * Page 1, lines 39-64; claims 1,7 * | 1 | |

|  |  |  | TECHNICAL FIELDS SEARCHED (Int. Cl.4) |
|---|---|---|---|
|  |  |  | G 01 N |

The present search report has been drawn up for all claims

| Place of search | Date of completion of the search | Examiner |
|---|---|---|
| THE HAGUE | 30-05-1989 | KOUZELIS D. |